# EUROPÄISCHE PATENTANMELDUNG

(11) **EP 4 169 489 A1**
(43) Veröffentlichungstag der Anmeldung: **26.04.2023**
(21) Anmeldenummer: 22202684.1
(22) Anmeldetag: 20.10.2022
(51) Int. Cl.: A61F 2/91

(54) **STENT**

(30) Priorität: 22.10.2021 DE 102021127510
(71) Anmelder: optimed medizinische Instrumente GmbH, 76275 Ettlingen (DE)
(72) Erfinder: Wille, Thomas, 01968 Senftenberg (DE); Wack, Thilo, 66636 Tholey (DE); Schmidt, Fabian, 76137 Karlsruhe (DE); Zipse, Achim, 76532 Baden-Baden (DE)
(74) Vertreter: Manitz Finsterwald Patent- und Rechtsanwaltspartnerschaft mbB

(57) **Zusammenfassung**

Die vorliegende Erfindung betrifft einen Stent zur transluminalen Implantation in Hohlorgane, insbesondere in Blutgefäße, Harnleiter, Speiseröhren, Colon, Duodenum, Luftwege oder Gallenwege, mit einem zumindest im Wesentlichen röhrenförmigen Körper, welcher sich entlang einer Längsrichtung erstreckt und der von einem komprimierten Zustand mit einem ersten Querschnittsdurchmesser in einen expandierten Zustand mit einem vergrößerten zweiten Querschnittsdurchmesser überführbar ist, wobei der Stent einen Stentkörper aus einem biostabilen Material umfasst, dadurch gekennzeichnet, dass der Stentkörper mehrere, insbesondere voneinander separate, bevorzugt ringförmige, Stentabschnitte umfasst, und der Stent eine Stützstruktur aufweist, welche die Stentabschnitte miteinander verbindet, wobei die Stützstruktur aus einem bioresorbierbaren Material geformt ist oder ein bioresorbierbares Material umfasst.

## Beschreibung

Die vorliegende Erfindung betrifft einen Stent zur transluminalen Implantation in Hohlorgane, insbesondere in Blutgefäße, Harnleiter, Speiseröhren, Colon, Duodenum, Luftwege oder Gallenwege, mit einem zumindest im Wesentlichen röhrenförmigen Körper, welcher sich entlang einer Längsrichtung erstreckt. Der Stent ist von einem komprimierten Zustand mit einem ersten Querschnittsdurchmesser in einen expandierten Zustand mit einem, im Vergleich zum ersten Querschnittsdurchmesser, vergrößerten zweiten Querschnittsdurchmesser überführbar. Der Stent umfasst einen Stentkörper aus einem biostabilen Material.

Stents werden zur Behandlung von krankhaft veränderten Hohlorganen eingesetzt, beispielsweise wenn sich die Hohlorgane verengt haben (Stenose). Ein anderes Anwendungsgebiet ist die Behandlung von Aneurysmen.

Zur Behandlung wird der Stent im komprimierten Zustand über einen Einführkatheter an die zu behandelnde Stelle innerhalb des Hohlorgans eingeführt, wo der Stent durch Dilatation oder durch Selbstexpansion auf einen Durchmesser, der beispielsweise dem Durchmesser des gesunden Hohlorgans entspricht, expandiert wird, so dass eine Stützwirkung des Hohlorgans, insbesondere einer Gefäßwand, erreicht wird.

Stents sollen üblicherweise eine hohe radiale Aufstellkraft bereitstellen, um eine ausreichend große Stützwirkung z.B. für ein Blutgefäß zu besitzen. Zudem soll der Stent in der Lage sein, sich an Verformungen des Hohlorgans anzupassen, die beispielsweise durch Bewegungen des Patienten entstehen. Aus diesem Grund soll der Stent flexibel ausgebildet sein, um Verformungen in der Längsrichtung zu gestatten.

Zudem ist es wünschenswert, den Stent auf einfache Weise und ortsgenau im Hohlorgan platzieren zu können.

Es ist daher die der Erfindung zugrundeliegende Aufgabe, einen verbesserten Stent bereitzustellen, der insbesondere den vorstehend genannten Aspekten Rechnung trägt.

Diese Aufgabe wird durch einen Stent gemäß Anspruch 1 gelöst.

Der erfindungsgemäße Stent zeichnet sich dadurch aus, dass der Stentkörper mehrere, insbesondere voneinander separate, bevorzugt ringförmige, Stentabschnitte umfasst. Zudem umfasst der erfindungsgemäße Stent eine Stützstruktur, welche die ringförmigen Stentabschnitte miteinander verbindet, wobei die Stützstruktur aus einem bioresorbierbaren Material geformt ist oder ein bioresorbierbares Material umfasst.

Das bioresorbierbare Material wird nach dem Einsetzen in das Hohlorgan abgebaut bzw. resorbiert, so dass nach einer gewissen Zeitdauer lediglich der Stentkörper aus dem biostabilen Material (dauerhaft) im Hohlorgan verbleibt. Die Erfindung setzt also auf der Erkenntnis auf, dass durch die Stützstruktur aus dem bioresorbierbaren Material die Stentabschnitte des Stentkörpers miteinander verbunden und auf diese Weise z.B. aneinander befestigt werden können, um das Einsetzen des Stentkörpers in das Hohlorgan zu erleichtern. Nach dem Auflösen bzw. der Resorption des bioresorbierbaren Materials fällt dann die Verbindung der Stentabschnitte durch die Stützstruktur weg, so dass sich z.B. die Flexibilität des Stents entlang der Längsrichtung vergrößern kann.

Bei der Stützstruktur kann es sich also um eine lediglich temporär im Körper verbleibende Struktur handeln, die vorteilhaft während des Einsetzens des Stents in das Hohlorgan ist, allerdings nicht zum dauerhaften Verbleib im Hohlorgan bestimmt ist.

Bevorzugt sind die Stentabschnitte voneinander separat. Dies bedeutet, die einzelnen Stentabschnitte sind nicht durch biostabiles Material des Stentkörpers miteinander verbunden, sondern werden z.B. lediglich durch die Stützstruktur miteinander verbunden. Durch die separaten Stentabschnitte ergibt sich nach Wegfall der Stützstruktur eine besonders hohe Flexibilität entlang der Längsrichtung. Überdies können die Stentabschnitte ringförmig ausgebildet sein, wobei zwischen den ringförmigen Stentabschnitten beispielsweise jeweils ein gleicher Abstand vorhanden sein kann. Alternativ oder zusätzlich sind aber auch beliebige andere Formen möglich, beispielsweise mit abgeschrägten und/oder unregelmäßig geformten Stentabschnitten.

Die einzelnen Stentabschnitte sind insbesondere nacheinander entlang der Längsrichtung aufgereiht angeordnet und weisen bevorzugt eine gemeinsame Achse auf, die sich in der Längsrichtung erstreckt.

Grundsätzlich weist der Stent einen im Wesentlichen röhrenförmigen Körper auf. Dies bedeutet, die Länge des Stents in der Längsrichtung ist bevorzugt größer (z.B. zumindest zweimal, fünfmal, zehnmal oder dreißigmal größer) als der Querschnittsdurchmesser des Stents. Im expandierten Zustand kann der Stent ein leeres, durchgängiges Lumen/Volumen in seinem Inneren aufweisen, durch das z.B. ein Blutfluss möglich ist. Der Stent kann über seine gesamte Länge in der Längsrichtung einen im expandierten Zustand im Wesentlichen gleichbleibenden Querschnittsdurchmesser aufweisen. Alternativ kann sich der Querschnittsdurchmesser über die Länge des Stents auch verändern, beispielsweise kontinuierlich abnehmen, um einer Durchmesserabnahme des Hohlorgans Rechnung zu tragen.

Bei dem Stent kann es sich um einen selbstexpandierenden Stent handeln, welcher ohne aktive Krafteinwirkung sich selbst von dem komprimierten Zustand in den expandierten Zustand überführt.

Alternativ kann der Stent auch nicht-selbstexpandierend sein und beispielsweise ballonexpandierbar sein. Im Inneren des Stents kann dann ein Ballon angebracht werden, wobei der Ballon unter Druck aufgepumpt wird, sich vergrößert und bei dieser Vergrößerung den Stent von dem komprimierten Zustand in den expandierten Zustand drängt und überführt ("Ballondilatation").

Bei dem biostabilen Material kann es sich beispielsweise um eine Nickel-Titan Legierung (Nitinol) handeln. Bevorzugt ist der Stentkörper aus einem eine Form speichernden Material gebildet ("shape memory"), das ab einer Grenztemperatur die gespeicherte Form einnimmt. Neben der genannten Nickel-Titan Legierung kann der Stentkörper auch als Kobalt-Chrom, Kobalt-Nickel oder Platin-Chrom Legierungen ausbildet sein oder solche Legierungen umfassen. Weiterhin sind auch andere geeignete Metalle und/oder Metall-Legierungen und/oder Metall-Werkstoffe möglich.

Bei dem Stent kann es sich insbesondere um einen "covered stent" handeln, welcher z.B. von einem Stoffmaterial umgeben ist. Der Stent kann auch einen Stentgraft umfassen. Der Stentgraft ist insbesondere eine künstliche Gefäßwand, die beispielsweise bei der Behandlung von Aneurysmen eingesetzt werden kann. Bei dem hierin beschriebenen Stent kann es sich aber auch um einen "bare stent" handeln, also einen Stent ohne Stentgraft oder Stoffmaterial.

Insbesondere umfasst die Stützstruktur mehrere separate Teile, d.h. die Stützstruktur bildet bevorzugt kein einzelnes zusammenhängendes Gebilde.

Der Stentkörper kann eine Vielzahl von Zellen umfassen, welche von von dem Stentkörper gebildeten Umrandungselementen (z.B. sogenannten "Struts") definiert werden. Die Umrandungselemente können entstehen, indem beim Schneiden des Stentkörpers aus insbesondere röhrenförmigem Material, Material entfernt wird, wobei die Umrandungselemente oder Stege bestehen bleiben. Die Umrandungselemente sind bevorzugt fest miteinander verbunden und insbesondere einstückig. Der Stentkörper umfasst dementsprechend zum Beispiel kein geflochtenes Material.

Eine Zelle kann durch einen Verbindungsabschnitt oder mehrere Verbindungsabschnitte mit einer oder mehreren anderen Zellen verbunden sein. Eine Zelle umfasst die gesamte Aussparung sowie ihre jeweiligen Umrandungselemente, wobei die Verbindungsabschnitte zu den Umrandungselementen gehören.

Bevorzugt bilden die Zellen des Stentkörpers ein konvexes Polygon und weisen insbesondere eine Rautenform auf. Alternativ können die Zellen auch rund, kreisförmig oder elliptisch ausgebildet sein. Die vorstehend genannten Formen der Zellen können sich insbesondere ergeben, wenn die Zellen auf eine Ebene gelegt oder gedrückt werden (die sogenannte Abwicklung). Bei einem konvexen Polygon weisen alle Innenwinkel jeweils einen Winkel von ≤180° auf. Solche Zellen können auch geschlossene Zellen ("closed cells") genannt werden. Durch die Form eines konvexen Polygons und insbesondere durch die Rautenform oder auch durch eine elliptische Form kann sich eine hohe Aufstellkraft und damit eine hohe Stützwirkung des Stents ergeben.

Insbesondere weisen die meisten oder alle Zellen die Form eines konvexen Polygons, eine Rautenform, eine Ringform, eine elliptische Form und/oder eine Kreisform auf.

Alternativ oder zusätzlich können Zellen des Stentkörpers auch ein konkaves Polygon bilden. Bei dem konkaven Polygon kann zumindest ein Innenwinkel einen Winkel von >180° aufweisen. Beispielsweise kann zumindest ein Teil der Umrandungselemente einer jeweiligen Zelle eine Zickzack-Form aufweisen. Durch solche Zickzack-Zellen oder allgemein durch Zellen mit der Form eines konkaven Polygons kann die Flexibilität des Stents weiter gesteigert werden. Es können auch die meisten oder alle Zellen die Form eines konkaven Polygons aufweisen.

Weiterhin ist es möglich, dass die Mehrheit oder alle Zellen des Stentkörpers dieselbe oder eine ähnliche Form aufweisen. Von einer ähnlichen Form kann insbesondere dann gesprochen werden, wenn die Umrandungselemente zweier Zellen beim Aufeinanderliegen einen maximalen Versatz aufweisen, der 10%, 20% oder 30% der Länge der größten Ausdehnung der Zelle in einer Raumrichtung nicht überschreitet.

Bevorzugt umfasst zumindest einer der Stentabschnitte eine Reihe von Zellen, die in Umfangsrichtung des Stents aufeinander folgen und vorzugsweise einen geschlossenen, in Umfangsrichtung des Stents umlaufenden Ring bilden. Der Ring weist senkrecht zur Längsrichtung des Stents vorzugsweise einen durch die Zellen des Rings gebildeten ringförmig geschlossenen Querschnitt auf. Durch einen solchen in Umfangsrichtung umlaufenden und bevorzugt geschlossenen Ring kann in dem Stentabschnitt eine optimale radiale Aufstellkraft und Stützwirkung des Stents bzw. des Stentkörpers geschaffen werden. Ein Stentabschnitt kann prinzipiell durch beliebige Zellen gebildet sein. Insbesondere kann ein ringförmiger Stentabschnitt genau eine einzige Reihe oder genau zwei oder drei miteinander verbundene Reihen von Zellen aufweisen. Durch zwei oder drei miteinander verbundene Reihen von Zellen verlängert sich der Stentabschnitt in der Längsrichtung, bietet aber auch eine erhöhte Aufstellkraft.

Vorteilhafte Weiterbildungen der Erfindung sind der Beschreibung, den Zeichnungen sowie den Unteransprüchen zu entnehmen.

Gemäß einer ersten Ausführungsform ist die Stützstruktur ausgebildet, die Stentabschnitte zueinander in einer definierten Relativposition zu halten. Die Stützstruktur befestigt also die Stentabschnitte, so dass deren Abstand und/oder Orientierung im expandierten und/oder komprimierten Zustand (zumindest ohne größere Krafteinwirkung von außen) bestehen bleiben. Ein gleichbleibender Abstand und/oder Orientierung ermöglicht ein verbessertes Einsetzen an einer sehr exakt bestimmbaren Zielposition. Während des Resorbierens der Stützstruktur im Hohlorgan erfolgt in der Regel ein gewisses Einwachsen der Stentabschnitte, so dass die Stentabschnitte dann auch ohne die Stützstruktur an ihrer jeweiligen Position verbleiben. Wie bereits erwähnt, erhöht sich aber ohne die Stützstruktur bevorzugt die Flexibilität entlang der Längsrichtung. Alternativ oder zusätzlich kann sich die Flexibilität des Stentkörpers grundsätzlich verbessern, so kann die longitudinale Dehnbarkeit und/oder Stauchbarkeit erhöht werden und/oder die Torsionsfähigkeit kann sich verbessern, wobei aber keine relevante Minderung der radialen Aufstellkraft, d.h. der Stützwirkung, eintritt.

Insbesondere kann unter der Verbindung bzw. Befestigung der Stentabschnitte durch die Stützstruktur auch verstanden werden, dass Relativbewegungen von Stützstruktur und Stentkörper oder Stentabschnitten weiterhin möglich sind, allerdings ein unabsichtliches Lösen von Stützstruktur und Stentkörper verhindert wird.

Gemäß einer weiteren Ausführungsform ist die Stützstruktur zumindest im Wesentlichen an der Außenseite des Stentkörpers angeordnet. Die Außenseite des Stentkörpers liegt beim Einsatz im Hohlorgan zumindest bereichsweise am Gewebe des Hohlorgans an. Insbesondere kann mehr als 50%, mehr als 80% oder mehr als 95% des Materials der Stützstruktur an der Außenseite und/oder außerhalb des Stentkörpers liegen. Bevorzugt ragt die Stützstruktur nicht über eine Innenseite des Stentkörpers in das freie Lumen im Inneren des Stents, um so eine Beeinträchtigung z.B. des Blutflusses durch das freie Lumen zu vermeiden.

Die Teile der Stützstruktur sind also insbesondere derart angeordnet, dass sie gegen die Wandung des Hohlorgans gedrückt werden oder zumindest im Bereich der Wandung des Hohlorgans zu liegen kommen, so dass beim Resorptionsvorgang vermieden wird, dass Fragmente der Stützstruktur z.B. in den Blutstrom gelangen und weggespült werden.

Gemäß einer weiteren Ausführungsform umfasst die Stützstruktur mehrere Schienen, welche zumindest im Wesentlichen parallel zu der Längsrichtung verlaufen. Die Schienen können bevorzugt an Verbindungsabschnitten der Zellen des Stentkörpers befestigt sein. Die Schienen sind weiter bevorzugt jeweils gerade ausgebildet und verlaufen z.B. an der Außenseite des Stentkörpers. Durch die Schienen können die notwendigen Kräfte zum Einführen und/oder Entlassen des Stents in/aus einem Katheter eines Einführbestecks verringert werden. Sowohl die Vorbereitung des Einführvorgangs als auch das Entlassen des Stents im Hohlorgan werden dadurch vereinfacht. Die Schienen erzeugen somit einen Vorteil beim Einsetzen des Stents, lösen sich dann aufgrund des bioresorbierbaren Materials auf und behindern auf Dauer die Flexibilität des Stents nicht.

Alternativ oder zusätzlich können zumindest manche Schienen auch einen spiralförmigen Verlauf entlang der Längsrichtung aufweisen, d.h. zumindest teilweise um eine von dem Stent gebildete Achse umlaufen.

Gemäß einer weiteren Ausführungsform sind die Schienen gleichmäßig in Umfangsrichtung des röhrenförmigen Körpers verteilt. Dies bedeutet, dass die Schienen im Querschnitt gesehen gleichmäßig beispielsweise entlang des Umfangs des Stentkörpers angeordnet sind. Von einer zentralen Achse des Stentkörper aus gesehen kann dann zumindest z.B. alle 30°, alle 45° oder alle 60° eine Schiene angeordnet sein.

Gemäß einer weiteren Ausführungsform weisen die Schienen eine gleiche Länge in der Längsrichtung auf. Die Länge der Schienen kann beispielsweise der Länge des Stentkörpers entsprechen. Die Schienen können auch kürzer als der Stentkörper ausgebildet sein und beispielsweise nur 30, 40, 50, 60 oder 70% der Länge des Stentkörpers in Längsrichtung aufweisen. Alternativ oder zusätzlich können zumindest manche der Schienen auch länger als der Stentkörper ausgebildet sein.

Gemäß einer weiteren Ausführungsform weisen die Schienen in der Längsrichtung gesehen verschiedene Positionen auf. Die Schienen können also verschiedene Anfangs- und/oder Endpositionen in der Längsrichtung aufweisen. Beispielsweise können sich jeweils zwei Schienen entlang der Längsrichtung nur teilweise überlappen. Auch ist es möglich, dass die Schienen einen Abstand zu den Enden des Stentkörpers aufweisen oder über die Enden des Stentkörpers hinausragen.

Gemäß einer weiteren Ausführungsform umfasst zumindest eine der Schienen zumindest ein Federelement, welches eine erhöhte Flexibilität im Vergleich zur Flexibilität der Schiene im Bereich außerhalb des Federelements besitzt, wobei das Federelement bevorzugt zwischen zwei Stentabschnitten angeordnet ist. Weiter bevorzugt ist das Federelement mittig zwischen zwei Stentabschnitten angeordnet. Durch das Federelement wird die Biegbarkeit bzw. Flexibilität des Stents insbesondere in der Längsrichtung erhöht, da durch die erhöhte Flexibilität des Federelements eine gewisse Beweglichkeit der Stentabschnitte zueinander ermöglicht werden kann. Das Federelement umfasst bevorzugt einen verjüngten Bereich und/oder eine Zickzackform und/oder eine Mäanderform, wobei durch den verjüngten Bereich und/oder die Zickzackform und/oder die Mäanderform die erhöhte Flexibilität des Federelements erzielt wird. Insbesondere durch die Positionierung zwischen den Stentabschnitten sind die Stentabschnitte dann, wie vorstehend ausgeführt, flexibler zueinander bewegbar.

Gemäß einer weiteren Ausführungsform ist die Stützstruktur mittels Formschluss und/oder Kraftschluss an dem Stentkörper befestigt. Insbesondere erfolgt die Befestigung der Stützstruktur an dem Stentkörper ausschließlich mittels Formschluss und/oder Kraftschluss. Der Formschluss und/oder Kraftschluss erfolgt bevorzugt zudem ausschließlich mit Material des Stentkörpers und der Stützstruktur, es wird also kein zusätzliches Material beispielsweise für ein Kleben, Löten, Schweißen und dergleichen benötigt.

Durch die Verbindung von Stützstruktur und Stentkörper mittels Formschluss und/oder Kraftschluss wird es ermöglicht, das biostabile Material mit dem bioresorbierbaren Material zu verbinden, obwohl beispielsweise Nitinol praktisch nicht mit einem bioresorbierbaren Material wie z.B. Zink, verschweißbar ist. Durch die hierin beschriebenen Befestigungen der Stützstruktur am Stentkörper ist dann aber dennoch ein sinnvoller Einsatz im Hohlorgan möglich.

Die Verbindung von Stützstruktur und Stentkörper mittels Formschluss und/oder Kraftschluss kann insbesondere derart erfolgen, dass im Stentkörper eine Aussparung mit einer Hinterschneidung vorgesehen ist, wobei die Stützstruktur in die Hinterschneidung hineinragt. Beispielsweise kann die Aussparung mit der Hinterschneidung im Querschnitt gesehen etwa trapezförmig (z.B. als gleichschenkliges Trapez) geformt sein, wobei das Trapez an seiner kürzeren Grundseite offen ausgebildet sein kann, wodurch die Hinterschneidung im Bereich der längeren Grundseite entsteht.

Das Material Stützstruktur kann in den Bereich der Aussparung mit der Hinterschneidung eingeführt und dort erhitzt werden, z.B. um es kurzzeitig zu verflüssigen. Dadurch kann das Material der Stützstruktur im Bereich der Hinterschneidung an dem Stentkörper anliegen und auf diese Weise einen Formschluss und gegebenenfalls auch einen Kraftschluss herstellen.

Gemäß einer weiteren Ausführungsform weist der Stentkörper zur Befestigung der Stützstruktur an dem Stentkörper einen Befestigungsvorsprung auf, um welchen die Stützstruktur zumindest zum Teil umlaufend ausgebildet ist. Bevorzugt kann die Stützstruktur auch vollständig um den Befestigungsvorsprung umlaufen. Es handelt sich somit um eine formschlüssige Verbindung von Stentkörper und Stützstruktur. Die Stützstruktur beschreibt beispielsweise einen Bogen (beim teilweisen Umlaufen) oder eine Schlaufe (beim vollständigen Umlaufen). Es handelt sich somit um eine formschlüssige Verbindung von Stützstruktur und Stentkörper.

Bevorzugt kann die Stützstruktur im Herstellungsprozess des Stents im Bereich des Befestigungsvorsprungs plattgedrückt werden, um beispielsweise plan mit der Innenseite des Stentkörpers zu sein und nicht in das Lumen des Stentkörpers hineinzuragen.

Gemäß einer weiteren Ausführungsform ist der Befestigungsvorsprung in einer Aussparung des Stentkörpers angeordnet und/oder ragt aus der Aussparung heraus. Die Aussparung kann im Querschnitt beispielsweise kreisförmig oder elliptisch geformt sein und dementsprechend eine Öse bilden, in der der Befestigungsvorsprung angeordnet ist und/oder aus der der Befestigungsvorsprung herausragt. Der Befestigungsvorsprung entspringt insbesondere aus der Innenwand der Aussparung und ragt (zunächst) in die Aussparung hinein. Die Stützstruktur kann sich dann beim teilweisen oder vollständigen Umlaufen um den Befestigungsvorsprung gegen die Innenwand der Aussparung abstützen, wodurch eine sichere Verbindung zwischen Stützstruktur und Stentkörper entsteht.

Der Befestigungsvorsprung kann beispielsweise gebogen ausgebildet sein und, insbesondere nur, zum Teil aus der Aussparung herausragen. Der Befestigungsvorsprung ragt bevorzugt aus der Außenseite des Stentkörpers heraus, um das Lumen im Inneren des Stentkörpers nicht zu beeinträchtigen.

Gemäß einer weiteren Ausführungsform ist der Stentkörper zur Befestigung der Stützstruktur mit der Stützstruktur verhakt, insbesondere mittels zweier ineinandergreifender Befestigungsringe, wobei zumindest einer der Befestigungsringe offen ausgebildet ist. Die Stützstruktur kann beispielsweise einen oder mehrere geschlossene Befestigungsringe aufweisen, wohingegen der Stentkörper ein oder mehrere offene Befestigungsringe umfasst. Die Befestigungsringe können dann wie Kettenglieder miteinander verbunden werden. Alternativ können auch die offenen Befestigungsringe an der Stützstruktur und die geschlossenen Befestigungsringe am Stentkörper vorgesehen sein. Die Befestigungsringe sind bevorzugt Ringscheiben, allerdings sind auch andere Formen möglich, beispielsweise oval, eckig, löffelförmig (d.h. gebogen, insbesondere mit demselben Radius wie der Stentkörper). Die Form der Befestigungsringe kann grundsätzlich beliebig sein, solange ein Befestigungsring des Stentkörpers in einen Befestigungsring der Stützstruktur eingreifen kann.

Bei einem offenen Befestigungsring kann ein Schlitz vorgesehen sein, welcher breiter als die Dicke des Materials des geschlossenen Befestigungsrings ist. Allerdings kann der Schlitz eine maximale Breite von zweimal, dreimal oder fünfmal der Dicke des Materials des Befestigungsrings aufweisen.

Bei der Befestigung durch die Befestigungsringe handelt es sich ebenfalls weder um eine formschlüssige Verbindung. Allgemein kann der Stentkörper mit der Stützstruktur also über Haken und Ösen miteinander verbunden sein.

Gemäß einer weiteren Ausführungsform wird ein Verhaken von Stentkörper und Stützstruktur mittels eines durch eine Öffnung geführten Widerhakens erreicht. Bei der Öffnung kann es sich insbesondere um einen geschlossenen Befestigungsring handeln, wie oben beschrieben. Der geschlossene Befestigungsring kann in diesem Fall bevorzugt an den Stentkörper angebracht sein. Der Widerhaken kann aus bioresorbierbarem Material geformt sein und umfasst einen Stab mit zumindest zwei an der Spitze des Stabes angebrachten rückwärts gerichteten Hakenabschnitten. Die Hakenabschnitte können gemeinsam eine V-Form definieren. Bei der Herstellung des Stents können die Hakenabschnitte nacheinander durch die Öffnung geführt werden, wobei danach ein Herausziehen der Hakenabschnitte verhindert wird. Durch den Widerhaken an der Stützstruktur kann somit eine Befestigung der Stützstruktur am Stentkörper erreicht werden.

Gemäß einer weiteren Ausführungsform umfasst die Stützstruktur mehrere Zylindersegmente im Inneren des Stentkörpers. Die Zylindersegmente liegen bevorzugt an der Innenwand des Stentkörpers an. Die Zylindersegmente entstehen durch ein Schneiden eines Zylinders entlang der Zylinderachse. Die Zylindersegmente können im Querschnitt die Form von Kreissektoren oder Kreisringabschnitten aufweisen. Die Kreisringabschnitte können zusammengesetzt insbesondere einen Hohlzylinder bilden.

Gemäß einer weiteren Ausführungsform bilden die Zylindersegmente im komprimierten Zustand des Stents einen Zylinder oder Hohlzylinder. Beim Übergang in den expandierten Zustand entfernen sich die Zylindersegmente dann voneinander und bilden, insbesondere innenliegende, an dem Stentkörper angebrachte Schienen. Die Zylindersegmente können mittels der hierin genannten Befestigungsmöglichkeiten an dem Stentkörper befestigt sein.

Alternativ oder zusätzlich zu einem innenliegenden Hohlzylinder ist es auch möglich, dass die Stützstruktur einen außenliegenden Hohlzylinder bzw. ein außenliegendes Rohr um den Stentkörper herum bildet, insbesondere im komprimierten Zustand.

Insbesondere kann die innenliegende Stützstruktur ein anderes Degradationsverhalten aufweisen als die außenliegende Stützstruktur, beispielsweise kann die innenliegende Stützstruktur schneller biodegradierbar sein als die außenliegende Stützstruktur. So kann die innenliegende Stützstruktur z.B. innerhalb von Minuten oder Stunden vollständig degradierbar sein, wohingegen die außenliegende Stützstruktur für die vollständige Degradation Tage oder Wochen benötigen kann.

Gemäß einer weiteren Ausführungsform umfasst die Stützstruktur mehrere Aussparungen oder Vertiefungen, in welchen Stentabschnitte des Stentkörpers zu liegen kommen. In diesem Fall kann die Stützstruktur insbesondere außenliegend sein, d.h. an der Außenseite des Stentkörpers angeordnet sein. Die Aussparungen können auch als Positionierungs-Aussparungen bezeichnet werden. Die Aussparungen/Vertiefungen können für die Stentabschnitte angepasst sein, so dass zwischen den Stentabschnitten dann Verdickungen der Stützstruktur vorhanden sind. Die Aussparungen oder Vertiefungen halten die Stentabschnitte und damit den Stentkörper in ihrer Position. Durch die Aussparungen oder Vertiefungen kann somit ebenfalls eine genaue Positionierung der einzelnen Stentabschnitte beim Einsetzen in das Hohlorgan erzielt werden.

Gemäß einer weiteren Ausführungsform sind zumindest zwei Stentabschnitte des Stentkörpers durch aus Material des Stentkörpers gebildeten Verbindungselementen miteinander verbunden, wobei an den Verbindungselementen Teile der Stützstruktur angebracht sind, um die Verbindungselemente zu verstärken. In dieser Ausführungsform sind die Stentabschnitte zumindest zum Teil nicht separat voneinander ausgebildet. Zur Verstärkung der Verbindungselemente mittels des bioresorbierbaren Materials können die Verbindungselemente beispielsweise mit dem bioresorbierbaren Material überzogen sein. Alternativ oder zusätzlich können die Verbindungselemente z.B. eine Nut oder eine andere Vertiefung aufweisen, in die das bioresorbierbare Material der Stützstruktur eingebracht ist. Beim Einsetzen des Stents in das Hohlorgan bewirkt das bioresorbierbare Material dann eine erhöhte Steifigkeit der Verbindungselemente, was wiederum gute Positioniereigenschaften nach sich zieht. Nach der Resorption des bioresorbierbaren Materials sind die Verbindungselemente dann durch den Wegfall des zusätzlichen Materials flexibler, wodurch sich die Flexibilität des Stents dann vorteilhaft erhöht.

Gemäß einer weiteren Ausführungsform weisen die Verbindungselemente zumindest bereichsweise ein dünneres und/oder verjüngtes Material auf. Das Material ist insbesondere im Vergleich zu den Stentabschnitten des Stentkörpers dünner und/oder verjüngt. Das dünnere und/oder verjüngte Material bezieht sich dabei nur auf das biostabile Material des Stentkörpers. Dünner bedeutet beispielsweise im Querschnitt weniger Fläche und/oder Material als im Querschnitt z.B. durch eine Strebe in einem Stentabschnitt. Zusammen mit dem Material der Stützstruktur kann das Verbindungselement genauso dick oder gar dicker sein als das biostabile Material im Bereich der Stentabschnitte.

Gemäß einer weiteren Ausführungsform drückt die Stützstruktur in zumindest einer Befestigungsaussparung des Stentkörpers gegen den Stentkörper, um die Stützstruktur an den Stentkörper mittels Kraftschluss zu befestigen. Bevorzugt kann die Stützstruktur bereichsweise mit dem Stentkörper verpresst sein. Die Befestigungsaussparung kann beispielsweise ein Loch oder Durchgangsloch in dem Stentkörper sein, in welches das bioresorbierbare Material der Stützstruktur eingepresst ist. Das Einpressen kann beispielsweise durch einen Pressdorn erfolgen, wie unten noch genauer erläutert. Im Ergebnis kann das bioresorbierbare Material der Stützstruktur allseitig gegen die Innenwand der Befestigungsaussparung drücken, wobei zentral in der Befestigungsaussparung ebenfalls eine Aussparung in dem bioresorbierbaren Material verbleibt.

Gemäß einer weiteren Ausführungsform ist der Stentkörper aus einem eine Form speichernden biostabilen Material gebildet, das ab einer Grenztemperatur die gespeicherte Form einnimmt, wobei das biostabile Material beispielsweise Nitinol ist oder umfasst, wie oben bereits erläutert.

Zudem kann der Stent zur einfacheren Positionierung unter Röntgenbeobachtung mehrere Röntgenmarker, insbesondere aus Tantal umfassen. Die Röntgenmarker können beispielsweise an den in Längsrichtung gesehenen Enden des Stentkörpers und/oder der Stützstruktur befestigt sein. Röntgenmarker an der Stützstruktur können insbesondere durch Materialverdickungen des bioresorbierbaren Materials gebildet sein. Bevorzugt erstreckt sich in Längsrichtung von zumindest einem Ende des Stentkörpers zumindest ein Röntgenmarker weg, insbesondere in Form einer Öse, wobei der Röntgenmarker eine asymmetrische Form aufweist. Ein Marker kann ein Abschnitt des Stentkörpers sein, der eine erhöhte Röntgendichtigkeit aufweist, d.h. in einem Röntgenbild besonders gut sichtbar ist. Insbesondere kann es sich bei dem Marker um eine Öse handeln, die beispielsweise mit dem vorgenannten Tantal gefüllt oder bedeckt ist.

Gemäß einer weiteren Ausführungsform umfasst das bioresorbierbare Material Zink. Bevorzugt besteht das bioresorbierbare Material aus Zink und Silber oder umfasst Zink und Silber. Insbesondere enthält das bioresorbierbare Material 90,0 bis 99,95 Massen-% Zink und 0,05 bis 10,0 Massen-% Silber. Ein solches bioresorbierbares Material kann in der Blutbahn beispielsweise innerhalb weniger Wochen vom Körper abgebaut werden.

Alternativ kann das bioresorbierbare Material auch einen polymeren Werkstoff umfassen oder aus einem solchen bestehen, beispielsweise aus Poly-Milchsäure (PLA) oder Poly-L-Milchsäure (PLLA). Das bioresorbierbare Material kann auch eine Magnesiumlegierung umfassen oder aus einer Magnesiumlegierung bestehen. Durch die oben beschriebene Verwendung von Zink oder einer Zinklegierung kann die Röntgensichtbarkeit des Stents im Vergleich zu PLA, PLLA oder Magnesiumlegierungen aber gesteigert werden. Das bioresorbierbare Material kann auch als biodegradierbares Material bezeichnet werden.

Es sind auch Kombinationen verschiedener bioresorbierbarer Materialien möglich. Ebenfalls kann die Stützstruktur ein oder mehrere verschiedene bioresorbierbare Materialien und ein oder mehrere röntgensichtbare Materialien umfassen.

Insbesondere kann der Stentkörper und/oder die Stützstruktur auch mit einem Wirkstoff versetzt oder beschichtet sein. Ein solcher Wirkstoff kann antiproliferativ wirken, um ein Zuwuchern des Stents mit Gewebe zu vermeiden. Beispielsweise können als Wirkstoff Anti-Proliferative der Limus-Gruppe, der Statine, der P2Y12-Antagonisten oder der Thrombinantagonisten verwendet werden.

Weiterer Gegenstand der Erfindung ist ein Stent-System mit einem Stent, der hierin beschriebenen Art und einem Katheter, in welchem der Stent im komprimierten Zustand aufgenommen ist oder aufnehmbar ist. Der Stent ist im Katheter bevorzugt von einer Hülle umgeben. Beim Einführen des Stents in den Katheter, d.h. beim Vorbereiten des Stent-Systems, kann insbesondere durch Schienen der Stützstruktur die notwendige Kraft zum Einführen in den Katheter reduziert werden. Ebenfalls kann die zum Entlassen aus dem Katheter bzw. der Hülle notwendige Kraft reduziert werden, was ein einfacheres Handling des Stent-Systems ermöglicht. Bevorzugt kann der Katheter an einer Innenwand Vertiefungen aufweisen, in denen die Schienen des Stents oder allgemein die Stützstruktur zu liegen kommt, um den Stent auf einfache Weise im Katheter positionieren zu können.

Der Katheter kann Teil eines sogenannten Einführbestecks sein, wobei das Einführbesteck insbesondere Handhabungsmittel zum Bewegen und Entlassen des Stents im Hohlorgan aufweist.

Insbesondere kann der Katheter ausgebildet sein, die Stentabschnitte beim Entlassen enger zusammenzuziehen, um das Entlassen zu vereinfachen und insbesondere die zum Entlassen benötigte Kraft zu reduzieren. Alternativ oder zusätzlich ist es möglich, dass der Katheter ausgebildet ist, beim Entlassen des Stents ein Verdrehen des Stents und insbesondere des Stentkörpers zu bewirken, so dass die Zellen des Stents nach Art von Zahnrädern ineinander greifen, d.h. dass jeweils eine Spitze einer Zelle in eine Ausbuchtung zwischen zwei Zellen hineinragt ("peak to valley"). Auf diese Weise kann eine durchgängigere Oberfläche des Stents entstehen, durch welche ein Drehen des Katheters um wenige Grad im Wechsel nach rechts und links vermieden werden kann (was sonst notwendig sein kann, wenn die Spitzen von Zellen benachbarter Stentabschnitte genau aufeinander gerichtet sind).

Alternativ oder zusätzlich kann der Katheter auch ausgebildet sein, einen Abstand der Stentabschnitte zueinander beim Entlassen einstellbar zu gestalten, z.B. in drei Schritten (dicht, normal, weit). Eine solche Abstandsänderung kann durch die Elastizität der Stützstruktur ermöglicht werden.

Das Zusammenziehen und/oder Verdrehen der Stentabschnitte kann z.B. durch Halten des Stents, insbesondere an den Enden des Stents, mittels zweier separater Haltemechanismen des Katheters erfolgen, wobei die zwei Haltemechanismen gegeneinander bewegt und/oder verdreht werden.

Weiterer Gegenstand der Erfindung ist ein Verfahren zur Herstellung eines Stents mit einem zumindest im Wesentlichen röhrenförmigen Körper, welcher sich entlang einer Längsrichtung erstreckt und der von einem komprimierten Zustand mit einem ersten Querschnittsdurchmesser in einen expandierten Zustand mit einem vergrößerten zweiten Querschnittsdurchmesser überführbar ist, wobei der Stent einen Stentkörper aus einem biostabilen Material umfasst, wobei der Stentkörper mehrere, insbesondere voneinander separate, bevorzugt ringförmige, Stentabschnitte umfasst, und der Stent eine Stützstruktur aufweist, welche die ringförmigen Stentabschnitte miteinander verbindet, wobei die Stützstruktur aus einem bioresorbierbaren Material geformt ist oder ein bioresorbierbares Material umfasst, wobei bei dem Verfahren eine Kraft auf die schon am Stentkörper anliegende Stützstruktur ausgeübt wird, um eine Verformung der Stützstruktur zu bewirken. Bei der Verformung kann es sich beispielsweise um ein Plattdrücken einer Schlaufe der Stützstruktur handeln, um in den Innenraum ragende Teile der Stützstruktur aus dem Innenraum herauszudrücken.

Gemäß einer Ausführungsform des Verfahrens erfolgt beim Ausüben der Kraft ein Verpressen der Stützstruktur mit dem Stentkörper, um die Stützstruktur an dem Stentkörper mittels Kraftschluss zu befestigen. Das Verpressen erfolgt bevorzugt in einer Befestigungsaussparung.

Allgemein gesprochen kann die Stützstruktur an einer oder mehreren Stellen mit dem Stentkörper verpresst sein, wobei die Stützstruktur beispielsweise in einem verpressten Bereich über Material des Stentkörpers übergestülpt und dann verpresst wird.

Gemäß einer weiteren Ausführungsform des Verfahrens wird während des Verpressens ein Innenrohr als Gegenlager in den Stentkörper eingeführt. Das Verpressen kann beispielsweise durch einen Pressdorn mit einer Kegelform erfolgen. Während des Verpressens kann im Inneren des Stentkörpers ein Innenrohr als Gegenlager eingeführt sein, so dass der Stentkörper durch den Druck des Pressdorns nicht kollabiert. Das Innenrohr kann eine Aussparung aufweisen, durch welche der Pressdorn beim Verpressen in das Innere des Innenrohrs geschoben werden kann. Durch den Pressdorn kann die Stützstruktur ringförmig und/oder allseitig an Wandungen der Befestigungsaussparung angepresst werden, wobei insbesondere ein Bereich ohne Material der Stützstruktur mittig in der Befestigungsaussparung entsteht. Der Bereich ohne Material entsteht nach Entfernen des Pressdorns, dort wo sich der Pressdorn beim Verpressen befunden hat.

Durch das Verpressen von Stützstruktur und Stentkörper kann eine haltbare Verbindung zwischen dem biostabilen Material und dem bioresorbierbaren Material geschaffen werden.

Für das erfindungsgemäße Stent-System und das erfindungsgemäße Verfahren gelten die zum erfindungsgemäßen Stent getroffenen Aussagen entsprechend. Dies gilt insbesondere hinsichtlich Vorteilen und Ausführungsformen. Zudem versteht es sich, dass sämtliche hierin genannten Ausführungsformen miteinander kombinierbar sind, sofern nicht explizit etwas Gegenteiliges angegeben ist.

Nachfolgend wird die Erfindung rein beispielhaft unter Bezugnahme auf die Zeichnungen beschrieben. Es zeigen:
- Fig. 1: schematisch einen Stent mit einem Stentkörper und einer Stützstruktur gemäß einer ersten Ausführungsform;
- Fig. 2: schematisch die Zellen eines Stents und Schienen der Stützstruktur gemäß einer zweiten Ausführungsform;
- Fig. 3: schematisch die Zellen eines Stents und Schienen der Stützstruktur gemäß einer dritten Ausführungsform;
- Fig. 4: schematisch die Verbindung der Stützstruktur mit dem Stentkörper gemäß einer vierten Ausführungsform;
- Fig. 5: schematisch die Befestigung der Stützstruktur mit Zellen des Stentkörpers gemäß einer fünften Ausführungsform;
- Fig. 6: schematisch die Verbindung der Stützstruktur mit dem Stentkörper gemäß einer sechsten Ausführungsform;
- Fig. 7: einen Stentkörper und eine Stützstruktur, welche als in Zylindersegmente geteilter Hohlzylinder im Inneren des Stentkörpers ausgebildet ist (siebte Ausführungsform);
- Fig. 8: schematisch Verbindungselemente zwischen Stentabschnitten, welche durch bioresorbierbares Material verstärkt sind (achte Ausführungsform); und
- Fig. 9: eine Stützstruktur gemäß einer neunten Ausführungsform, wobei die Stützstruktur Vertiefungen umfasst, in welchen Stentabschnitte zu liegen kommen.

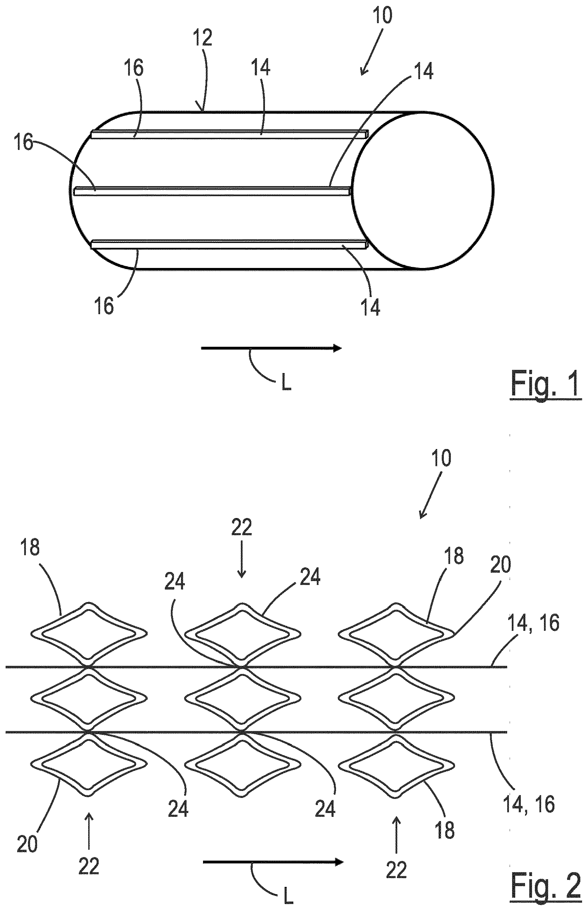

Fig. 1 zeigt einen Stent 10 einer ersten Ausführungsform im expandierten Zustand. Der Stent 10 weist eine röhrenförmige Gestalt auf und erstreckt sich entlang einer Längsrichtung L. Der Stent umfasst einen röhrenförmigen Stentkörper 12, wobei der Stentkörper 12 mit einer Stützstruktur 14 verbunden ist. In der Fig. 1 ist die Stützstruktur 14 in Form von mehreren Schienen 16 gezeigt.

Der Stentkörper 12 ist aus einem biostabilen Material, beispielsweise Nitinol, gebildet. Die Stützstruktur 14 hingegen besteht aus einem bioresorbierbaren Material, beispielsweise einer Zink-Legierung.

Für die weiteren Ausführungsformen werden im Wesentlichen nur die Unterschiede zur Ausführungsform von Fig. 1 erläutert.

Fig. 2 zeigt eine zweite Ausführungsform des Stents 10. Fig. 2 zeigt dabei einen Ausschnitt aus einer Abwicklung des Stents 10. Der Stentkörper 12 ist aus Zellen 18 geformt. Die Zellen 18 weisen jeweils eine Rautenform auf und sind aus stegartigen Umrandungselementen 20 gebildet.

Der Stent 10 kann jeweils noch zusätzliche Zellen 18 aufweisen, welche in den Figuren nicht gezeigt sind.

Die in Fig. 2 oben gezeigten Zellen 18 sind jeweils mit den in Fig. 2 unten gezeigten Zellen 18 verbunden, um so die Röhrenform des Stentkörpers 12 zu schaffen. Die Zellen 18 des Stents 10 bilden jeweils voneinander separate ringförmige Stentabschnitte 22. Jeder Stentabschnitt 22 weist eine Reihe von Zellen 18 auf, die in Umfangsrichtung miteinander verbunden sind. Die Zellen 18 eines Stentabschnitts 22 sind jeweils über zwei Verbindungsabschnitte 24 mit den in Umfangsrichtung benachbarten Zellen 18 verbunden.

Die Schienen 16 sind jeweils im Bereich der Verbindungsabschnitte 24 mit dem Stentkörper 12 gekoppelt. Die Schienen 16 sind länglich und gerade ausgebildet und erstrecken sich über die gesamte Länge des Stentkörpers 12. Die einzelnen Stentabschnitte 22 selbst sind lediglich über die Schienen 16 der Stützstruktur 14 miteinander verbunden.

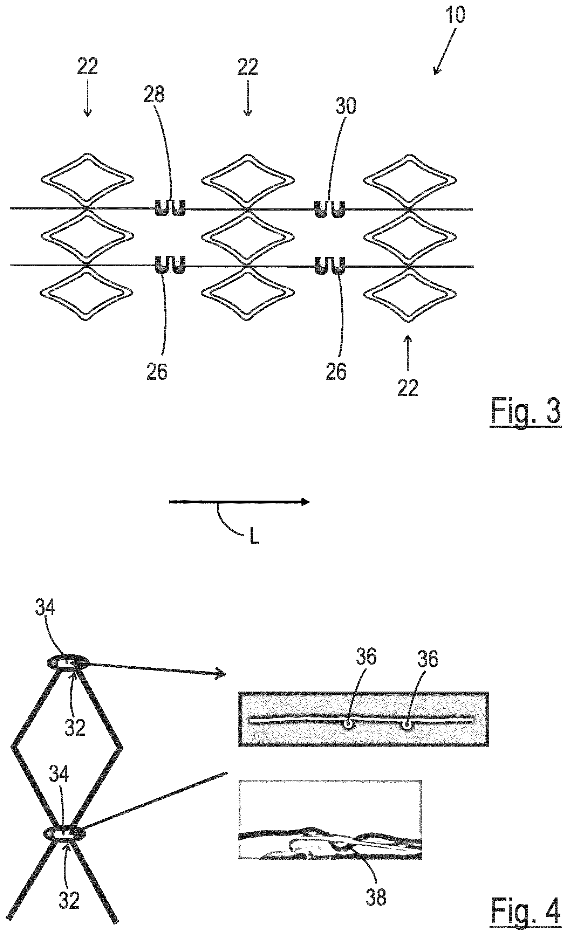

Fig. 3 zeigt eine dritte Ausführungsform des Stents 10. Die dritte Ausführungsform unterscheidet sich von der Ausführungsform gemäß Fig. 2 dadurch, dass die Schienen 16 jeweils zwischen zwei Stentabschnitten 22 ein Federelement 26 aufweisen. Das Federelement 26 umfasst eine Verjüngung 28, welche Teil einer mäanderförmigen Struktur 30 ist. Die Federelemente 26 gestatten eine gewisse Beweglichkeit der Stentabschnitte 22 untereinander.

Fig. 4 zeigt eine vierte Ausführungsform, wobei eine Möglichkeit zur Verbindung der Stützstruktur 14 mit dem Stentkörper 12 dargestellt ist. Fig. 4 zeigt in Verbindungsabschnitte 24 eingebrachte Aussparungen 32, wobei in die Aussparungen 32 ein Befestigungsvorsprung 34 hineinragt. Um die Befestigungsvorsprünge 34 können dann Schlaufen 36 oder Bögen 38 der Stützstruktur 14 gelegt werden, um die Stützstruktur 14 an dem Stentkörper 12 zu befestigen.

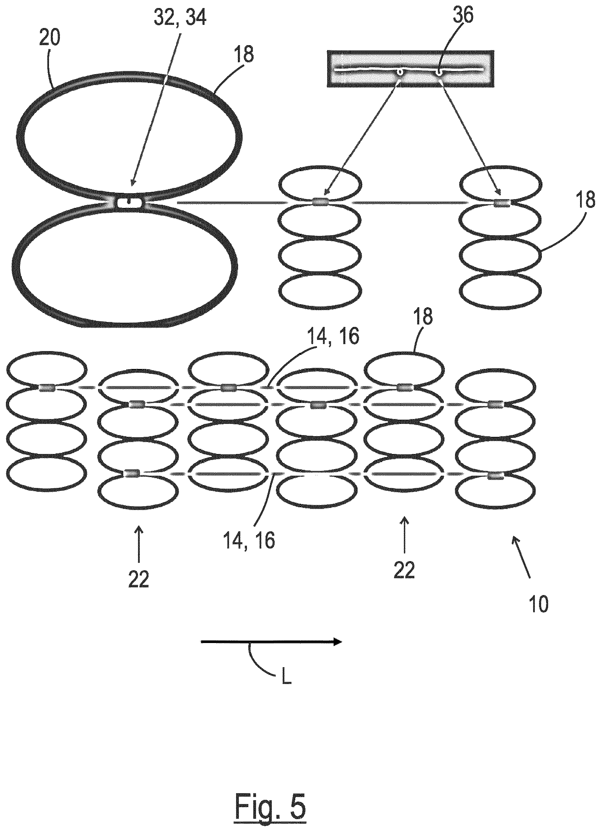

Die in Fig. 5 gezeigte fünfte Ausführungsform umfasst ebenfalls Befestigungsvorsprünge 34, um welche Schlaufen 36 der Stützstruktur 14 gelegt werden. Im Unterschied zur Ausführungsform von Fig. 4 umfasst die fünfte Ausführungsform elliptische Zellen 18. Zudem zeigt die fünfte Ausführungsform Schienen 16, welche jeweils nur an (in der Längsrichtung L gesehen) jedem zweiten Stentabschnitt 22 befestigt sind. Zudem sind die Schienen 16 kürzer als die Länge des Stentkörpers 12 ausgebildet.

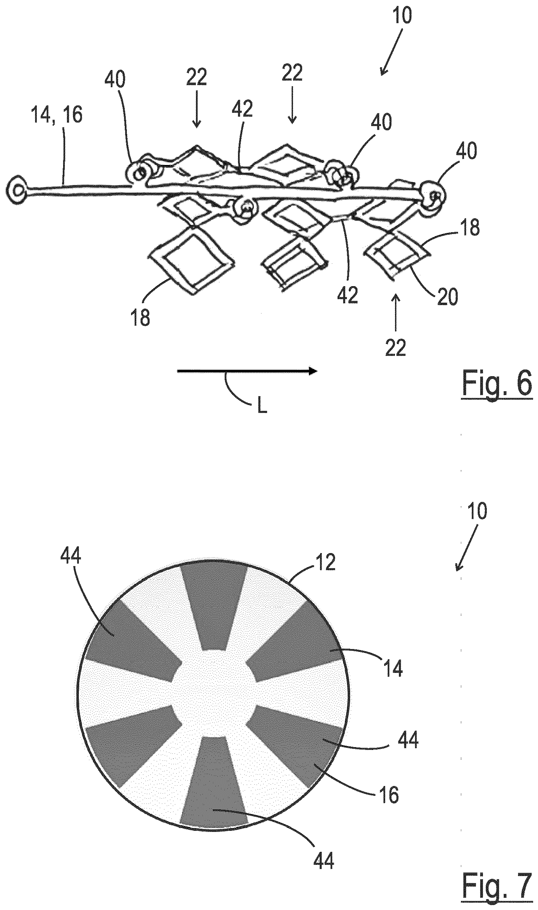

Fig. 6 zeigt eine sechste Ausführungsform des Stents 10. Bei der sechsten Ausführungsform ist die Stützstruktur 14 mittels Befestigungsringen 40 mit dem Stentkörper 12 verbunden. Sowohl der Stentkörper 12 als auch die Stützstruktur 14 weisen dabei Befestigungsringe 40 auf. Die Befestigungsringe 14 des Stentkörpers 12 können dabei offen sein und einen (nicht gezeigten) Schlitz aufweisen, durch welchen der Befestigungsring 40 der Stützstruktur 14 in dem Befestigungsring 40 des Stentkörpers 12 eingreifen kann. Dementsprechend können die Befestigungsringe 40 der Stützstruktur 14 geschlossene Befestigungsringe 40 sein.

Bei der Ausführungsform gemäß Fig. 6 können die einzelnen Stentabschnitte 22 auch durch Material des Stentkörpers 12, genauer gesagt durch Verbindungselemente 42, miteinander verbunden sein. Die Verbindungselemente 42 erstrecken sich jeweils von einem Ende einer rautenförmigen Zelle 18 eines Stentabschnitts 22 bis zu einem Ende einer Zelle 18 eines direkt benachbarten Stentabschnitts 22.

Fig. 7 zeigt eine siebte Ausführungsform eines Stents 10 im expandierten Zustand. Fig. 7 zeigt dabei eine Ansicht in Richtung der Längsachse L. Innerhalb des Stentkörpers 12 umfasst die Stützstruktur 14 mehrere Zylindersegmente 44, welche im komprimierten Zustand einen Hohlzylinder bilden. Die Zylindersegmente 44 bilden innenliegende Schienen 16 innerhalb des Stentkörpers 12.

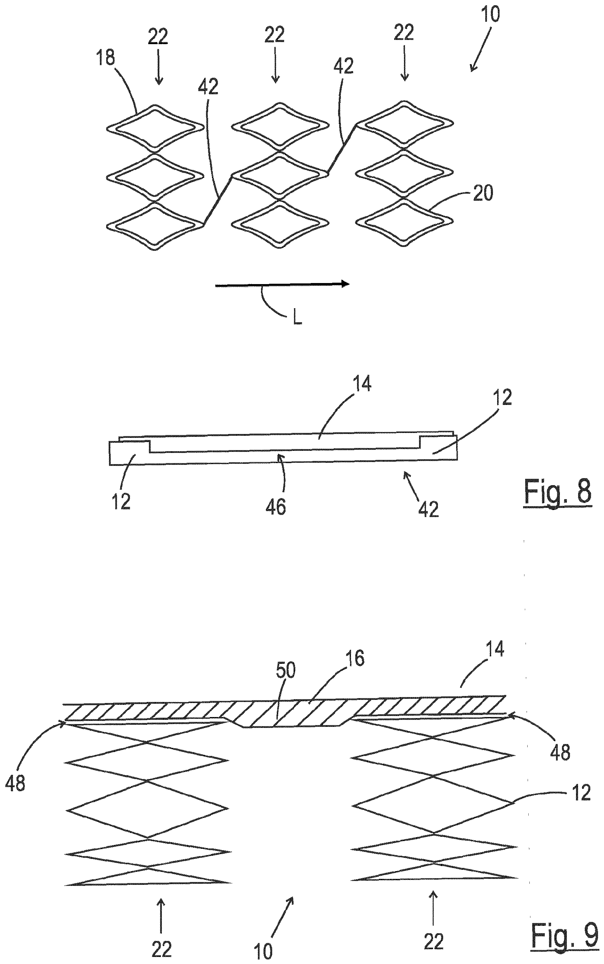

Fig. 8 zeigt eine achte Ausführungsform des Stents 10, bei welcher die Stentabschnitte 22 durch Verbindungselemente 42 verbunden sind. Im oberen Bereich der Fig. 8 ist hierzu ein Teil einer Abwicklung des Stents 10 gezeigt (Draufsicht). Im unteren Teil der Fig. 8 ist eine Seitenansicht auf ein Verbindungselement 42 dargestellt. Das Verbindungselement 42 ist aus biostabilem Material des Stentkörpers 12 geformt und wird durch bioresorbierbares Material der Stützstruktur 14 verstärkt. Das der Verstärkung dienende Material der Stützstruktur 14 ist in eine Aufnahme 46 des Verbindungselements 42 aufgenommen, wobei die Aufnahme 46 z.B. durch eine Aussparung in dem Verbindungselement 42 gebildet sein kann.

Fig. 9 zeigt eine neunte Ausführungsform des Stents 10, wobei eine schematische Seitenansicht des Stents 10 dargestellt ist. Der Stent 10 umfasst wiederum mehrere Stentabschnitte 22, die mit einer Stützstruktur 14 verbunden sind, wobei in Fig. 9 eine Schiene 16 der Stützstruktur 14 dargestellt ist. Die Schiene 16 umfasst mehrere Positionierungs-Aussparungen 48, in welchen die Stentabschnitte 22 zu liegen kommen und so relativ zueinander in vorgegebenen Positionen gehalten werden. Zwischen den Positionierungs-Aussparungen 48 ist eine Verdickung 50 der Schiene 16 vorgesehen.

Wie vorstehend ausgeführt, können die unterschiedlichen Ausführungsformen miteinander kombiniert werden. Beispielsweise können die Positionierungs-Aussparungen 48 auch in den Schienen 16 der vorangegangenen Ausführungsformen integriert sein. Ebenfalls ist es möglich, unterschiedliche Verbindungsmethoden zwischen dem Stentkörper 12 und der Stützstruktur 14 miteinander zu kombinieren.

Allen Ausführungsformen ist gemein, dass die Stützstruktur 14 eine erhöhte Stabilität des Stents 10 beim Einsetzen in ein Hohlorgan bewirkt. Die Stützstruktur 14 ist nach dem Einsetzen nur temporär vorhanden und wird resorbiert, wobei nach der Resorption der Vorteil einer erhöhten Flexibilität des Stents 10 gegeben ist.

### Bezugszeichenliste

- 10: Stent
- 12: Stentkörper
- 14: Stützstruktur
- 16: Schiene
- 18: Zelle
- 20: Umrandungselement
- 22: Stentabschnitt
- 24: Verbindungsabschnitt
- 26: Federelement
- 28: Verjüngung
- 30: mäanderförmige Struktur
- 32: Aussparung
- 34: Befestigungsvorsprung
- 36: Schlaufe
- 38: Bogen
- 40: Befestigungsring
- 42: Verbindungselement
- 44: Zylindersegment
- 46: Aufnahme
- 48: Positionierungs-Aussparung
- 50: Verdickung

## Patentansprüche

1. Stent (10) zur transluminalen Implantation in Hohlorgane, insbesondere in Blutgefäße, Harnleiter, Speiseröhren, Colon, Duodenum, Luftwege oder Gallenwege, mit einem zumindest im Wesentlichen röhrenförmigen Körper, welcher sich entlang einer Längsrichtung (L) erstreckt und der von einem komprimierten Zustand mit einem ersten Querschnittsdurchmesser in einen expandierten Zustand mit einem vergrößerten zweiten Querschnittsdurchmesser überführbar ist,
wobei der Stent (10) einen Stentkörper (12) aus einem biostabilen Material umfasst,
**dadurch gekennzeichnet, dass**
der Stentkörper (12) (12) mehrere, insbesondere voneinander separate, bevorzugt ringförmige, Stentabschnitte (22) umfasst, und
der Stent (10) eine Stützstruktur (14) aufweist, welche die Stentabschnitte (22) miteinander verbindet, wobei die Stützstruktur (14) aus einem bioresorbierbaren Material geformt ist oder ein bioresorbierbares Material umfasst.

2. Stent (10) nach Anspruch 1,
wobei die Stützstruktur (14) ausgebildet ist, die Stentabschnitte (22) zueinander in einer definierten Relativposition zu halten
und/oder
wobei die Stützstruktur (14) zumindest im Wesentlichen an der Außenseite des Stentkörpers (12) angeordnet ist.

3. Stent (10) nach zumindest einem der vorstehenden Ansprüche,
wobei die Stützstruktur (14) mehrere Schienen (16) umfasst, welche zumindest im Wesentlichen parallel zu der Längsrichtung verlaufen,
wobei bevorzugt die Schienen (16) gleichmäßig in Umfangsrichtung des röhrenförmigen Körpers verteilt sind und/oder eine gleiche Länge in der Längsrichtung aufweisen und/oder in der Längsrichtung verschiedene Positionen aufweisen.

4. Stent (10) nach zumindest Anspruch 3,
wobei zumindest eine der Schienen (16) zumindest ein Federelement (26) umfasst, welches eine erhöhte Flexibilität im Vergleich zur Flexibilität der Schiene (16) im Bereich außerhalb des Federelements (26) besitzt, wobei das Federelement (26) bevorzugt zwischen zwei ringförmigen Stentabschnitten (22) angeordnet ist, weiter bevorzugt mittig zwischen zwei ringförmigen Stentabschnitten (22).

5. Stent (10) nach zumindest einem der vorstehenden Ansprüche,
wobei die Stützstruktur (14) mittels Formschluss und/oder Kraftschluss an dem Stentkörper (12) befestigt ist.

6. Stent (10) nach zumindest einem der vorstehenden Ansprüche,
wobei der Stentkörper (12) zur Befestigung der Stützstruktur (14) an dem Stentkörper (12) einen Befestigungsvorsprung (34) aufweist, um welchen die Stützstruktur (14) zumindest zum Teil umlaufend ausgebildet ist,
wobei der Befestigungsvorsprung (34) inbesondere in einer Aussparung (32) des Stentkörpers (12) angeordnet ist und/oder aus der Aussparung (32) herausragt.

7. Stent (10) nach zumindest einem der vorstehenden Ansprüche,
wobei der Stentkörper (12) zur Befestigung der Stützstruktur (14) mit der Stützstruktur (14) verhakt ist, insbesondere mittels zweier ineinandergreifender Befestigungsringe (40), wobei zumindest einer der Befestigungsringe (40) offen ausgebildet ist,
wobei insbesondere ein Verhaken von Stentkörper (12) und Stützstruktur (14) mittels eines durch eine Öffnung geführten Widerhakens erreicht wird.

8. Stent (10) nach zumindest einem der Ansprüche 1, 2 und 4 bis 11,
wobei die Stützstruktur (14) mehrere Zylindersegmente (44) im Inneren des Stentkörpers (12) umfasst,
wobei die Zylindersegmente (44) im komprimierten Zustand des Stents insbesondere einen Zylinder oder Hohlzylinder bilden.

9. Stent (10) nach zumindest einem der vorstehenden Ansprüche,
wobei die Stützstruktur (14) mehrere Aussparungen (48) und/oder Vertiefungen umfasst, in welchen Stentabschnitte (22) des Stentkörpers (12) zu liegen kommen.

10. Stent (10) nach zumindest einem der vorstehenden Ansprüche,
wobei eine innenliegende Stützstruktur (14) ein anderes Degradationsverhalten aufweist als eine außenliegende Stützstruktur (14), beispielsweise ist die innenliegende Stützstruktur (14) schneller biodegradierbar als die außenliegende Stützstruktur (14).

11. Stent (10) nach zumindest einem der vorstehenden Ansprüche,
wobei zumindest zwei Stentabschnitte (22) des Stentkörpers (12) durch aus Material des Stentkörpers (12) gebildeten Verbindungselementen (42) miteinander verbunden sind, wobei an den Verbindungselementen (42) Teile der Stützstruktur (14) angebracht sind, um die Verbindungselemente (42) zu verstärken.

12. Stent (10) nach zumindest einem der vorstehenden Ansprüche, wobei die Stützstruktur (14) in zumindest einer Befestigungsaussparung des Stentkörpers (12) gegen den Stentkörper (12) drückt, um die Stützstruktur (14) an dem Stentkörper (12) mittels Kraftschluss zu befestigen.

13. Stent-System mit einem Stent (10) gemäß zumindest einem der vorstehenden Ansprüche und einem Katheter, in welchem der Stent (10) im komprimierten Zustand aufgenommen ist oder aufnehmbar ist,
wobei der Katheter an einer Innenwand bevorzugt Vertiefungen aufweist, in denen die Stützstruktur des Stents (10) zu liegen kommt.

14. Stent-System nach Anspruch 21 oder 22,
wobei der Katheter ausgebildet ist, einen Abstand der Stentabschnitte (22) zueinander beim Entlassen einzustellen oder zu verändern und/oder die Stentabschnitte (22) beim Entlassen gegeneinander zu verdrehen.

15. Verfahren zur Herstellung eines Stents mit einem zumindest im Wesentlichen röhrenförmigen Körper, welcher sich entlang einer Längsrichtung (L) erstreckt und der von einem komprimierten Zustand mit einem ersten Querschnittsdurchmesser in einen expandierten Zustand mit einem vergrößerten zweiten Querschnittsdurchmesser überführbar ist,
wobei der Stent (10) einen Stentkörper (12) aus einem biostabilen Material umfasst,
wobei der Stentkörper (12) mehrere, insbesondere voneinander separate, bevorzugt ringförmige, Stentabschnitte (22) umfasst, und
der Stent (10) eine Stützstruktur (14) aufweist, welche die ringförmigen Stentabschnitte (22) miteinander verbindet, wobei die Stützstruktur (14) aus einem bioresorbierbaren Material geformt ist oder ein bioresorbierbares Material umfasst,
wobei bei dem Verfahren eine Kraft auf die schon am Stentkörper (12) anliegende Stützstruktur (14) ausgeübt wird, um eine Verformung der Stützstruktur (14) zu bewirken.
